# FASCICULE DE BREVET EUROPEEN

(11) **EP 0 618 794 B1**
(45) Date de publication et mention de la délivrance du brevet: **08.11.1995**
(21) Numéro de dépôt: 93902365.1
(22) Date de dépôt: 22.12.1992
(51) Int. Cl.: A61K 7/42, A61K 7/00

(54) **UTILISATION DU METHYLSULFATE DE 4- (2-OXO-3-BORNYLIDENE)METHYL]PHENYLTRIMETHYLAMMONIUM DANS UNE COMPOSITION DE DEFORMATION PERMANENTE DES CHEVEUX POUR LA PROTECTION CONTRE LES AGRESSIONS ATMOSPHERIQUES, ET EN PARTICULIER CONTRE LA LUMIERE**
VERWENDUNG VON 4-[(2-OXO-3-BORNYLIDENE)METHYL]-FENYLTRIMETHYLAMMONIUM METHYLSULFAT IN EINER ZUSAMMENSETZUNG ZUR DAUERHAFTEN VERFORMUNG
USE OF 4- (2-OXO-3-BORNYLIDENE)METHYL]-PHENYLTRIMETHYLAMMONIUM METHYL SULPHATE COMPOSITION FOR PERMANENT HAIR SHAPING, AFFORDING PROTECTION FROM THE RAVAGES OF THE WEATHER, AND ESPECIALLY LIGHT

(30) Priorité: 23.12.1991 FR 9116019
(43) Date de publication de la demande: 12.10.1994
(73) Titulaire: L'OREAL, 75008 Paris (FR)
(72) Inventeur: DE LABBEY, Arnaud, F-93600 Aulnay-sous-Bois (FR); N'GUYEN, Ly Lan, F-94240 La Haye-les-Roses (FR)
(74) Mandataire: Casalonga, Axel
(86) Numéro de dépôt international: FR9201223
(87) Numéro de publication internationale: WO9312763

(56) Documents cités:
- EP-A- 0 329 032
- EP-A- 0 440 547
- FR-A- 2 199 971

## Description

La présente invention a pour objet l'utilisation du méthylsulfate de 4-[(2-oxo-3-bornylidène)méthyl]-phényltriméthylammonium dans une composition de déformation permanente des cheveux en tant qu'agent de protection de la kératine des cheveux contre les agressions atmosphériques, et en particulier contre la lumière, et un procédé de protection des propriétés mécaniques des cheveux soumis à l'action successive d'un traitement de déformation permanente et des agressions atmosphériques, en particulier la lumière, à l'aide de ce composé.

On sait depuis longtemps que la lumière agresse la kératine des cheveux. De nombreuses publications divulguent que la lumière naturelle détruit certains aminoacides des cheveux et qu'en altérant la fibre capillaire, elle en dégrade les propriétés mécaniques; par dégradation des propriétés mécaniques, en entend principalement la diminution du palier à 15% d'extension.

Le palier à 15% d'extension est le poids qu'il faut appliquer à un cheveu mouillé d'une longueur donnée pour l'allonger de 15%. Plus le poids est élevé, plus le cheveu est élastique et résistant.

Pour lutter contre l'agression des cheveux par la lumière, on a déjà proposé d'utiliser des substances susceptibles de filtrer les radiations lumineuses.

En tant qu'agents de protection des propriétés mécaniques des cheveux et essentiellement du palier à 15% d'extension au mouillé, la demanderesse a antérieurement proposé d'utiliser, dans les demandes de brevets EP 329032 et français 2 627 085 des agents filtrants tels que l'acide 4-(2-oxo-3-bornylidèneméthyl)benzène sulfonique ou ses sels et l'acide 2-hydroxy 4-méthoxybenzophénone-5-sulfonique ou ses sels.

Ces composés ont notamment été préconisés pour être appliqués avant ou après un shampooing, une coloration, une décoloration, une permanente ou un défrisage.

Il est par ailleurs connu d'utiliser des substances filtrantes substantives telles que des sels particuliers d'ammonium quaternaire d'acides carboxyliques décrits dans le brevet US 4 680 144, dans le but de prévenir l'éclaircissement de la couleur artificielle des cheveux à la lumière. Lesdites substances ont été préconisées sous forme de shampooings ou de sprays à appliquer après coloration.

Le brevet FR 2 509 989 cite des filtres solaires hydrosolubles pouvant être utilisés dans la phase aqueuse d'une composition cosmétique pour la protection de la peau et des cheveux contre le rayonnement UV, cette composition comprenant une phase huileuse séparée. Il n'y est fait aucune allusion au type de cheveux protégés ni aux propriétés mécaniques des cheveux.

Dans l'art antérieur, aucune substance filtrante n'a donc encore été préconisée pour être utilisée dans une composition de déformation permanente dans le but de préserver de la lumière les propriétés mécaniques des cheveux permanentés, et notamment leur élasticité.

Or, il est bien connu qu'un maximum de dégradation des propriétés mécaniques, et en particulier de l'élasticité, est observé dans le cas de cheveux soumis à une opération de déformation permanente puis agressés ensuite par la lumière. Ceci est en relation avec une fragilisation accrue du cheveu par l'action cumulée du réducteur et du fixateur.

Dans ce cas précis, aucune substance filtrante ne s'est encore révélée suffisamment efficace pour protéger le cheveu soumis à une opération de déformation permanente et à l'action successive de la lumière.

La demanderesse a maintenant découvert de façon surprenante, que le méthylsulfate de 4-[(2-oxo-3-bornylidène)méthyl]phényltriméthylammonium pouvait préserver efficacement les propriétés mécaniques des cheveux soumis à une opération de déformation permanente, notamment leur élasticité, contre la dégradation par les agressions atmosphériques, et en particulier la lumière, quand le composé actif susmentionné est introduit en solution aqueuse soit dans la phase réductrice, soit dans la phase oxydante dite de fixation ou en application intrapermanente, c'est-à-dire entre les deux étapes de réduction et de fixation.

Cette propriété a pu être mise en évidence par exposition en lumière naturelle (milieu ensoleillé) et en lumière artificielle (émetteur au xénon d'un appareil de vieillissement accéléré du type SUNTEST HANAU).

Le méthylsulfate de 4-[(2-oxo-3-bornylidène)méthyl]phényltriméthylammonium est décrit dans le brevet français 2 199 971 en tant que filtre UV pouvant être appliqué sur la peau ou incorporé dans les compositions cosmétiques contenant des constituants photosensibles.

La présente invention a donc pour objet l'utilisation du méthylsulfate de 4-[(2-oxo-3-bornylidène)méthyl]phényltriméthylammonium dans une composition de déformation permanente sous forme de solution aqueuse, en tant qu'agent de protection des propriétés mécaniques des cheveux et essentiellement de leur élasticité mesurée par le palier à 15% d'extension au mouillé, contre la dégradation provoquée par l'action successive d'une déformation permanente et des agressions atmosphériques, notamment la lumière.

Selon l'invention et avantageusement, on a constaté que cette protection s'avérait rémanente dans le temps même après un shampooing et qu'elle permettait en outre de colorer les cheveux permanentés ou défrisés dans des nuances présentant une meilleure résistance à la lumière.

Dans l'invention, on utilise le méthylsulfate de 4-[(2-oxo-3-bornylidène)méthyl]phényltriméthylammonium en des quantités comprises entre 0,05 et 15%, et de préférence comprises entre 1 et 10% en poids, par rapport au poids total de la composition de réduction ou de la composition de fixation ou encore de la composition intrapermanente.

Lorsque l'agent filtrant est utilisé dans la composition réductrice, on préfère utiliser l'agent filtrant et le réducteur en un mélange extemporané que l'on effectue au moment de l'emploi.

Les compositions de réduction et de fixation peuvent renfermer tous les ingrédients connus de l'art antérieur pour effectuer une opération de permanente ou de défrisage, tels qu'ils sont décrits par exemple dans Harry's Cosmeticology, 7e édition - 1982, p. 569-577.

Les compositions intrapermanentes sont des compositions essentiellement aqueuses, éventuellement épaissies. Elles peuvent renfermer, tout comme les compositions de réduction et de fixation, des adjuvants permettant d'améliorer l'état cosmétique des fibres capillaires, parmi lesquels on peut citer à titre d'exemples, des protéines naturelles végétales ou synthétiques pouvant être quaternisées, des acides aminés ou des polymères; elles peuvent aussi renfermer des agents renforçateurs de la fibre capillaire, parmi lesquels on peut citer par exemple des sels hydrosolubles, minéraux ou organiques, de métaux divalents tels que le chlorure, le sulfate, l'aspartate ou l'acétate de magnésium.

La présente invention vise également un procédé de protection des propriétés mécaniques, et essentiellement de l'élasticité mesurée par le palier à 15% d'extension au mouillé, des cheveux soumis à l'action successive d'un traitement de déformation permanente et des agressions atmosphériques, en particulier la lumière, consistant à appliquer sur les cheveux, une composition aqueuse réductrice ou de fixation ou dite "intrapermanente" utilisée entre les deux étapes de réduction et de fixation du traitement de déformation permanente, renfermant une quantité efficace de méthylsulfate de 4-[(2-oxo-3-bornylidène)méthyl]phényltriméthylammonium.

Les exemples suivants illustrent l'invention sans pour autant la limiter.

### EXEMPLE 1

On prépare une composition réductrice pour permanente des cheveux, de composition suivante :

| | |
|---|---|
| - Acide thioglycolique | **6,7** g |
| - Carbonate d'ammonium | **5,6** g |
| - Méthylsulfate de 4-[(2-oxo-3-bornylidène)méthyl])phényltriméthylammonium | **5,0** g |
| - Chlorure d'oléocétyl diméthylammonium | **1,5** g |
| - Ammoniaque | qs pH : **8,0** |
| - Parfum, stabilisant | qs |
| - Eau déminéralisée | qsp **100** g |

Cette composition est préparée extemporanément par addition de 5 g de méthylsulfate de 4-[(2-oxo-3-bornylidène)méthyl]phényltriméthylammonium au reste de la composition.

On applique cette composition sur des cheveux mouillés, préalablement enroulés sur des bigoudis. On laisse agir 15 minutes, puis on rince abondamment à l'eau.

On applique alors la composition oxydante suivante :

| | |
|---|---|
| - Peroxyde d'hydrogène en solution aqueuse à 200 volumes | **4,8** g |
| - Stabilisants : sulfate de 8-hydroxy quinoléine et phénacétine | **0,06** g |
| - Alcool oléique à 20 moles d'oxyde d'éthylène | **1,5** g |
| - Acide citrique | qs pH : **3** |
| - Eau déminéralisée | qsp **100** g |

On laisse agir la composition oxydante pendant 10 minutes. On rince à l'eau puis on enlève les bigoudis et on sèche les cheveux.

Les cheveux sont alors exposés 120 heures au SUNTEST à l'aide d'un appareil "SUNTEST HANAU". Cet appareil est constitué d'un émetteur au xénon et d'un système de filtres produisant un rayonnement correspondant, dans une très large mesure, au rayonnement solaire. Le rayonnement énergétique est de 585 W/m² environ dans le domaine de longueurs d'onde comprises entre 300 et 830 nm (rayonnement global).

La permanente ainsi réalisée permet, grâce au composé actif introduit dans sa phase de réduction, d'améliorer la valeur moyenne du palier à 15% d'extension au mouillé de manière très significative par rapport à des cheveux de même nature, permanentés de façon identique, sans le méthylsulfate de 4-[(2-oxo-3-bornylidène)méthyl]phényltriméthylammonium dans la phase de réduction.

### EXEMPLE 2

On prépare la composition réductrice suivante :

| | |
|---|---|
| - Acide thioglycolique | **9,3** g |
| - Monoéthanolamine | **3,1** g |
| - Carbonate d'ammonium | **3,0** g |
| - Mélange de cocamidopropylbétaïne et de laurate de glycéryle vendu sous la dénomination "Tégobétaïne HS" (GOLDSCHMIDT), en solution à 30% MA | **0,7** g MA |
| - Ammoniaque | qs pH : **8,3** |
| - Parfum, stabilisant | qs |
| - Eau déminéralisée | qsp **100** g |

On applique cette composition sur cheveux mouillés préalablement enroulés sur des bigoudis; on laisse agir 15 minutes puis on rince abondamment.

On applique alors la lotion intrapermanente suivante :

| | |
|---|---|
| - Chlorure de magnésium | **1,1** g |
| - Acide L-aspartique | **0,4** g |
| - L-isoleucine | **0,4** g |
| - Glycine | **0,4** g |
| - D.L Sérine | **0,4** g |
| - Protéine quaternisée résultant de la condensation de la cocamidopropyl-diméthylamine sur une protéine animale hydrolysée, dénommée dans le supplément de la 4ème édition (1991) du dictionnaire CTFA : Quaternium-76 Hydrolysed Collagen, vendue sous la dénomination commerciale "LEXEIN QX 3000" par la société INOLEX | **2,3** g |
| - Acide sorbique | **0,3** g |
| - Méthylsulfate de 4-[(2-oxo-3-bornylidène)méthyl] phényltriméthylammonium | **3,0** g |
| - Eau déminéralisée | qsp **100** g |

On laisse en contact pendant 5 minutes et on applique sans rincer la composition de fixation suivante :

| | |
|---|---|
| - Peroxyde d'hydrogène en solution aqueuse à 200 volumes | **4,8** g |
| - Stabilisants : sulfate de 8-hydroxy quinoléine et phénacétine | **0,06** g |
| - Chlorure de distéaryldiméthylammonium | **0,5** g |
| - Acide citrique | qs pH :**3** |
| - Eau déminéralisée | qsp **100** g |

On laisse poser 10 minutes. On rince à l'eau, on enlève les bigoudis et on sèche les cheveux.

On expose ensuite les cheveux 120 heures au SUNTEST, comme décrit à l'exemple 1.

Par rapport à des cheveux de même nature et permanentés de façon identique mais sans composé actif : méthylsulfate de 4-[(2-oxo-3- bornylidène)méthyl]phényltriméthylammonium dans la composition intrapermanente, on constate une amélioration appréciable de la valeur moyenne du palier à 15% d'extension au mouillé.

### EXEMPLE 3

On prépare la composition réductrice suivante :

| | |
|---|---|
| - Acide thioglycolique | **7,5** g |
| - Carbonate d'ammonium | **4,0** g |
| - Chlorure d'oléocétyldiméthylammonium | **1,2** g |
| - Ammoniaque | qs pH : **8,1** |
| - Parfum, stabilisant | qs |
| - Eau déminéralisée | qsp **100** g |

On applique cette composition sur des cheveux mouillés, préalablement enroulés sur des bigoudis. On laisse agir 15 minutes puis on rince abondamment à l'eau.

On applique ensuite pendant 10 minutes la composition de fixation suivante :

| | |
|---|---|
| - Peroxyde d'hydrogène en solution aqueuse à 200 volumes | **4,8** g |
| - Stabilisants : sulfate de 8-hydroxy quinoléine et phénacétine | **0,06** g |
| - Oxyde de lauryldiméthylamine | **0,7** g |
| - Méthylsulfate de 4-[(2-oxo-3-bornylidène)méthyl] phényltriméthylammonium | **3,0** g |
| - Eau déminéralisée | qsp **100** g |

On laisse agir pendant 10 minutes, on rince à l'eau et on sèche les cheveux.

On expose ensuite les cheveux 120 heures au SUNTEST, comme décrit à l'exemple 1.

Par rapport à des cheveux de même nature et permanentés de façon identique mais sans composé actif : méthylsulfate de 4-[(2-oxo-3-bornylidène)méthyl] phényltriméthylammonium dans la composition de fixation, on constate une amélioration appréciable de la valeur moyenne du palier à 15% d'extension au mouillé et une amélioration cosmétique de l'état des cheveux permanentés.

### EXEMPLE 4

On reproduit l'exemple 1 en procédant, après la permanente et avant l'exposition au SUNTEST, à une coloration des cheveux ainsi permanentés.

Par rapport à des cheveux de même nature, permanentés en l'absence du composé actif dans la phase de réduction, puis colorés avant de subir la même exposition, on observe une supériorité au niveau de la tenue des couleurs.

### EXEMPLE 5

On reproduit l'exemple 2, en conservant la composition réductrice et la composition de fixation, et en utilisant une lotion intrapermanente ayant une autre composition et qui comprend :

| | |
|---|---|
| - Asparate de magnésium | **2,0** g |
| - Acide L-aspartique | **0,5** g |
| - L-isoleucine | **0,5** g |
| - Glycine | **0,5** g |
| - D.L Sérine | **0,5** g |
| - Hydrolysat de kératine de laine (PM=1000) quaternisée, en solution aqueuse à 25% MA (PROMOIS WK-HQ vendu par SEIWA KASEI) | **1,5** g MA |
| - Acide sorbique | **0,3** g |
| - Méthylsulfate de 4-[(2-oxo-3-bornylidène)méthyl] phényltriméthylammonium | **4,0** g |
| - Eau déminéralisée | qsp **100** g |

Après exposition 120 heures au SUNTEST, on constate une amélioration notable de la valeur moyenne du palier à 15% d'extension au mouillé par rapport à des cheveux de même nature et permanentés de façon identique mais sans le composé actif.

## Revendications

1. Utilisation du méthylsulfate de 4-[(2-oxo-3-bornylidène)méthyl]-phényltriméthylammonium dans une composition de déformation permanente sous forme de solution aqueuse, pour préserver les propriétés mécaniques des cheveux, essentiellement leur élasticité mesurée par le palier à 15% d'extension au mouillé, de la dégradation provoquée par l'action successive d'une déformation permanente et des agressions atmosphériques, notamment la lumière.

2. Utilisation selon la revendication 1 du méthylsulfate de 4-[(2-oxo-3-bornylidène)méthyl]-phényltriméthylammonium dans une composition aqueuse de réduction pour la déformation permanente des cheveux.

3. Utilisation selon la revendication 1 du méthylsulfate de 4-[(2-oxo-3-bornylidène)méthyl]-phényltriméthylammonium, dans une composition aqueuse de fixation pour la déformation permanente des cheveux.

4. Utilisation selon la revendication 1 du méthylsulfate de 4-[(2-oxo-3-bornylidène)méthyl]-phényltriméthylammonium, dans une composition aqueuse intrapermanente utilisée entre les deux étapes de réduction et de fixation du traitement de déformation permanente des cheveux.

5. Utilisation selon l'une quelconque des revendications 1 à 4 du méthylsulfate de 4-[(2-oxo-3-bornylidène)méthyl]-phényltriméthylammonium en des quantités comprises entre 0,05 et 15% en poids, par rapport au poids total de la composition de réduction, de fixation ou de la composition intrapermanente.

6. Utilisation selon l'une quelconque des revendications 1 à 5 du méthylsulfate de 4-[(2-oxo-3-bornylidène)méthyl]-phényltriméthylammonium, en des quantités comprises entre 1 et 10% en poids, par rapport au poids total de la composition de réduction, de fixation ou de la composition intrapermanente.

7. Utilisation selon la revendication 2 du méthylsulfate de 4-[(2-oxo-3-bornylidène)méthyl]-phényltriméthylammonium dans une composition aqueuse réductrice pour la déformation permanente des cheveux, caractérisée par le fait que le méthylsulfate de 4-[(2-oxo-3-bornylidène)méthyl]-phényltriméthylammonium et le réducteur sont utilisés en un mélange extemporané que l'on effectue au moment de l'emploi.

8. Utilisation selon l'une quelconque des revendications 1 à 7 du méthylsulfate de 4-[(2-oxo-3-bornylidène)méthyl]-phényltriméthylammonium dans une composition aqueuse de déformation permanente renfermant des protéines naturelles végétales ou synthétiques pouvant être quaternisées, des acides aminés, des polymères et des sels hydrosolubles, minéraux ou organiques, de métaux divalents.

9. Procédé de protection des propriétés mécaniques des cheveux, et essentiellement de leur élasticité mesurée par le palier à 15% d'extension au mouillé, contre la dégradation provoquée par l'action successive d'une déformation permanente et des agressions atmosphériques, en particulier la lumière, caractérisé par le fait qu'il consiste à appliquer sur les cheveux, une quantité efficace de méthylsulfate de 4-[(2-oxo-3-bornylidène)méthyl]-phényltriméthylammonium, en solution dans une composition aqueuse de déformation permanente.

10. Procédé selon la revendication 9, caractérisé par le fait qu'il consiste à appliquer sur les cheveux, une quantité efficace de méthylsulfate de 4-[(2-oxo-3-bornylidène)méthyl]-phényltriméthylammonium, en solution dans une composition aqueuse réductrice pour la déformation permanente des cheveux.

11. Procédé selon la revendication 9, caractérisé par le fait qu'il consiste à appliquer sur les cheveux une quantité efficace de méthyl sulfate de 4-[(2-oxo-3-bornylidène)méthyl]-phényltriméthylammonium, en solution dans une composition aqueuse de fixation pour la déformation permanente des cheveux.

12. Procédé selon la revendication 9, caractérisé par le fait qu'il consiste à appliquer sur les cheveux, une quantité efficace de méthylsulfate de 4-[(2-oxo-3-bornylidène)méthyl]-phényltriméthylammonium, en solution dans une composition aqueuse intrapermanente utilisée entre les étapes de réduction et de fixation de la déformation permanente des cheveux.

13. Procédé selon l'une quelconque des revendications 9 à 12, caractérisé par le fait qu'il consiste à appliquer sur les cheveux 0,05 à 15% en poids de méthylsulfate de 4-[(2-oxo-3-bornylidène)méthyl]-phényltriméthylammonium, en solution dans une composition aqueuse de réduction, de fixation ou intrapermanente.

14. Procédé selon la revendication 13, caractérisé par le fait qu'elle consiste à appliquer sur les cheveux, 1 à 10% en poids de méthylsulfate de 4-[(2-oxo-3-bornylidène)méthyl]-phényltriméthylammonium, en solution dans une composition aqueuse de réduction, de fixation ou intrapermanente.

15. Procédé selon la revendication 10, caractérisé par le fait qu'on effectue un mélange extemporané au moment de l'emploi, du méthylsulfate de 4-[(2-oxo-3-bornylidène)méthyl]-phényltriméthylammonium, et du réducteur.

16. Procédé selon l'une quelconque des revendications 9 à 15, caractérisé par le fait qu'on introduit dans la composition aqueuse de déformation permanente, des protéines naturelles végétales ou synthétiques pouvant être quaternisées, des acides aminés, des polymères et des sels hydrosolubles, minéraux ou organiques, de métaux divalents.

## Claims

1. Use of 4-[(2-oxo-3-bornylidene)methyl]phenyltrimethylammonium methyl sulfate in a permanent shaping composition in the form of an aqueous solution for protecting the mechanical properties of hair, essentially its elasticity measured by the wet 15% extension stage, from the degradation brought about by the successive action of a permanent shaping and the ravages of the weather, in particular light.

2. Use according to Claim 1 of 4-[(2-oxo-3-bornylidene)methyl]phenyltrimethylammonium methyl sulfate in an aqueous reduction composition for the permanent shaping of hair.

3. Use according to Claim 1 of 4-[(2-oxo-3-bornylidene)methyl]phenyltrimethylammonium methyl sulfate in an aqueous fixing composition for the permanent shaping of hair.

4. Use according to Claim 1 of 4-[(2-oxo-3-bornylidene)methyl]phenyltrimethylammonium methyl sulfate in an aqueous intrapermanent composition used between the two steps of reduction and fixing in the treatment for the permanent shaping of hair.

5. Use according to any one of Claims 1 to 4 of 4-[(2-oxo-3-bornylidene)methyl]phenyltrimethylammonium methyl sulfate in amounts between 0.05 and 15% by weight, relative to the total weight of the reduction or fixing composition or of the intrapermanent composition.

6. Use according to any one of Claims 1 to 5 of 4-[(2-oxo-3-bornylidene)methyl]phenyltrimethylammonium methyl sulfate in amounts between 1 and 10% by weight, relative to the total weight of the reduction or fixing composition or of the intrapermanent composition.

7. Use according to Claim 2 of 4-[(2-oxo-3-bornylidene)methyl]phenyltrimethylammonium methyl sulfate in an aqueous reducing composition for the permanent shaping of hair, characterized in that 4-[(2-oxo-3-bornylidene)methyl]phenyltrimethylammonium methyl sulfate and the reducing agent are used in an extemporaneous mixture which is prepared at the time of use.

8. Use according to any one of Claims 1 to 7 of 4-[(2-oxo-3-bornylidene)methyl]phenyltrimethylammonium methyl sulfate in an aqueous permanent shaping composition containing natural plant or synthetic proteins which may be quaternized, amino acids, polymers and inorganic or organic water-soluble salts of divalent metals.

9. Process for protecting the mechanical properties of hair, and essentially its elasticity measured by the wet 15% extension stage, against the degradation brought about by the successive action of a permanent shaping and the ravages of the weather, in particular light, characterized in that it consists in applying to the hair an effective amount of 4-[(2-oxo-3-bornylidene)methyl]phenyltrimethylammonium methyl sulfate dissolved in an aqueous permanent shaping composition.

10. Process according to Claim 9, characterized in that it consists in applying to the hair an effective amount of 4-[(2-oxo-3-bornylidene)methyl]phenyltrimethylammonium methyl sulfate dissolved in an aqueous reducing composition for the permanent shaping of hair.

11. Process according to Claim 9, characterized in that it consists in applying to the hair an effective amount of 4-[(2-oxo-3-bornylidene)methyl]phenyltrimethylammonium methyl sulfate dissolved in an aqueous fixing composition for the permanent shaping of hair.

12. Process according to Claim 9, characterized in that it consists in applying to the hair an effective amount of 4-[(2-oxo-3-bornylidene)methyl]phenyltrimethylammonium methyl sulfate dissolved in an aqueous intrapermanent composition used between the reduction and fixing steps in the permanent shaping of hair.

13. Process according to any one of Claims 9 to 12, characterized in that it consists in applying to the hair 0.05 to 15% by weight of 4-[(2-oxo-3-bornylidene)methyl]phenyltrimethylammonium methyl sulfate dissolved in an aqueous reduction, fixing or intrapermanent composition.

14. Process according to Claim 13, characterized in that it consists in applying to the hair 1 to 10% by weight of 4-[(2-oxo-3-bornylidene)methyl]phenyltrimethylammonium methyl sulfate dissolved in an aqueous reduction, fixing or intrapermanent composition.

15. Process according to Claim 10, characterized in that an extemporaneous mixing of 4-[(2-oxo-3-bornylidene)methyl]phenyltrimethylammonium methyl sulfate and of the reducing agent is carried out at the time of use.

16. Process according to any one of Claims 9 to 15, characterized in that natural plant or synthetic proteins which may be quaternized, amino acids, polymers and inorganic or organic water-soluble salts of divalent metals are introduced into the aqueous permanent shaping composition.

## Patentansprüche

1. Verwendung von 4-((2-Oxo-3-bornyliden)methyl)phenyltrimethylammoniummethylsulfat in einer Zusammensetzung zur dauerhaften Verformung in Form einer wässrigen Lösung zur Bewahrung der mechanischen Eigenschaften der Haare, im wesentlichen von deren Elastizität, gemessen durch die auf befeuchtetes Haar für eine 15%-ige Dehnung anzuwendende Gewichtskraft, wobei die Haare vor einem Abbau geschützt werden, der durch die aufeinanderfolgende Einwirkung einer dauerhaften Verformung und von Angriffen aus der Atmosphäre, insbesondere von Licht, hervorgerufen wird.

2. Verwendung gemäß Anspruch 1 des 4-(2-Oxo-3-bornyliden)methyl)phenyltrimethylammoniummethylsulfats in einer wässrigen Zusammensetzung zur Reduktion für die dauerhafte Verformung von Haaren.

3. Verwendung gemäß Anspruch 1 des 4-((2-Oxo-3-bornyliden)methyl)phenyltrimethylammoniummethylsulfats in einer wässrigen Zusammensetzung zur Fixierung für die dauerhafte Verformung der Haare.

4. Verwendung gemäß Anspruch 1 des 4-((2-Oxo-3-bornyliden)methyl)phenyltrimethylammoniummethylsulfats in einer wässrigen Zusammensetzung als Zwischenbehandlung bei der Dauerwelle, die zwischen den beiden Stufen der Reduktion und Fixierung der Behandlung zur dauerhaften Verformung der Haare angewandt wird.

5. Verwendung gemäß jedem der Ansprüche 1 bis 4 des 4-((2-Oxo-3-bornyliden)methyl)phenyltrimethylammoniummethylsulfats in Mengen von 0,05 bis 15 Gew.%, bezogen auf das Gesamtgewicht der Zusammensetzung zur Reduktion, Fixierung oder Zwischenbehandlung der Dauerwelle.

6. Verwendung gemäß jedem der Ansprüche 1 bis 5, des 4-((2-Oxo-3-bornyliden)methyl)phenyltrimethylammoniummethylsulfats in Mengen von 1 bis 10 Gew.%, bezogen auf das Gesamtgewicht der Zusammensetzung zur Reduktion, Fixierung oder Zwischenbehandlung der Dauerwelle.

7. Verwendung gemäß Anspruch 2 des 4-((2-Oxo-3-bornyliden)methyl)phenyltrimethylammoniummethylsulfats in einer wässrigen reduzierenden Zusammensetzung für die dauerhafte Verformung der Haare,
dadurch **gekennzeichnet**, daß
das 4-((2-Oxo-3-bornyliden)methyl)phenyltrimethylammoniummethylsulfat und das Reduktionsmittel in einer unmittelbar zubereiteten Abmischung angewandt werden, wobei man die Abmischung zum Zeitpunkt der Anwendung durchführt.

8. Verwendung gemäß jedem der Ansprüche 1 bis 7 des 4-((2-Oxo-3-bornyliden)methyl)phenyltrimethylammoniummethylsulfats in einer wässrigen Zusammensetzung zur dauerhaften Verformung, die natürliche pflanzliche oder synthetische, gegebenenfalls quaternierte Proteine, Aminosäuren, Polymere und wasserlösliche, mineralische oder organische Salze zweiwertiger Metalle enthält.

9. Verfahren zum Schutz der mechanischen Eigenschaften der Haare, und insbesondere von deren Elastizität, gemessen durch die auf befeuchtetes Haar für eine 15%-ige Dehnung anzuwendende Gewichtskraft, vor dem Abbau, der durch die aufeinanderfolgende Einwirkung einer dauerhaften Verformung und von Angriffen aus der Atmosphäre, insbesondere von Licht, hervorgerufen wird,
dadurch **gekennzeichnet,** daß
man auf die Haare eine wirksame Menge von 4-((2-Oxo-3-bornyliden)methyl)phenyltrimethylammoniummethylsulfat in Lösung in einer wässrigen Zusammensetzung zur dauerhaften Verformung aufbringt.

10. Verfahren gemäß Anspruch 9,
dadurch **gekennzeichnet**, daß
man auf die Haare eine wirksame Menge des 4-((2-Oxo-3-bornyliden)methyl)phenyltrimethylammoniummethylsulfats n Lösung in einer wässrigen reduzierenden Zusammensetzung für die dauerhafte Verformung der Haare aufbringt.

11. Verfahren gemäß Anspruch 9,
dadurch **gekennzeichnet**, daß
man auf die Haare eine wirksame Menge des 4-((2-Oxo-3-bornyliden)methyl)phenyltrimethylammoniummethylsulfats in Lösung in einer wässrigen Zusammensetzung zur Fixierung für die dauerhafte Verformung der Haare aufbringt.

12. Verfahren gemäß Anspruch 9,
dadurch **gekennzeichnet,** daß
man auf die Haare eine wirksame Menge des 4-((2-Oxo-3-bornyliden)methyl)phenyltrimethylammoniummethylsulfats in Lösung in einer wässrigen Zusammensetzung zur Zwischenbehandlung bei der Dauerwelle aufbringt, die zwischen den Stufen der Reduktion und Fixierung der dauerhaften Verformung der Haare angewandt wird.

13. Verfahren gemäß jedem der Ansprüche 9 bis 12,
dadurch **gekennzeichnet,** daß
man auf die Haare 0,05 bis 15 Gew.% 4-((2-Oxo-3-bornyliden)methyl)phenyltrimethylammoniummethylsulfat in Lösung in einer wässrigen Zusammensetzung zur Reduktion, Fixierung oder Dauerwellenzwischenbehandlung aufbringt.

14. Verfahren gemäß Anspruch 13,
dadurch **gekennzeichnet,** daß
man auf die Haare 1 bis 10 Gew.% 4-((2-Oxo-3-bornyliden)methyl)phenyltrimethylammoniummethylsulfat in Lösung in einer wässrigen Zusammensetzung zur Reduktion, Fixierung oder Dauerwellenzwischenbehandlung aufbringt.

15. Verfahren gemäß Anspruch 10,
dadurch **gekennzeichnet,** daß
man eine unmittelbar zum Zeitpunkt der Anwendung zubereitete Mischung des 4-((2-Oxo-3-bornyliden)methyl)phenyltrimethylammoniummethylsulfats und des Reduktionsmittels durchführt.

16. Verfahren gemäß jedem der Ansprüche 9 bis 15,
dadurch **gekennzeichnet,** daß
man in die wässrige Zusammensetzung zur dauerhaften Verformung natürliche pflanzliche oder synthetische, gegebenenfalls quaternierte Proteine, Aminosäuren, Polymere und wasserlösliche, mineralische oder organische Salze zweiwertiger Metalle einbringt.
